# EUROPÄISCHE PATENTSCHRIFT

(11) **EP 1 417 346 B1**
(45) Veröffentlichungstag und Bekanntmachung des Hinweises auf die Patenterteilung: **26.11.2008**
(21) Anmeldenummer: 02760269.7
(22) Anmeldetag: 25.07.2002
(51) Int. Cl.: C12Q 1/68, C12Q 1/02, C12N 15/70

(54) **VERFAHREN ZUM IDENTIFIZIEREN VON MAKROCYCLISCHEN POLYKETIDEN**
METHOD FOR IDENTIFYING MACROCYCLIC POLYKETIDES
PROCEDE D'IDENTIFICATION DE POLYKETIDES MACROCYCLIQUES

(30) Priorität: 07.08.2001 DE 10138766
(43) Veröffentlichungstag der Anmeldung: 12.05.2004
(73) Patentinhaber: Bayer CropScience AG, 40789 Monheim (DE)
(72) Erfinder: EBERZ, Günther, 51519 Odenthal (DE); MÖHRLE, Volker, 50733 Köln (DE)
(86) Internationale Anmeldenummer: PCT/EP2002/008282
(87) Internationale Veröffentlichungsnummer: WO 2003/014386

(56) Entgegenhaltungen:
- WO-A-92/15687
- WO-A-94/13831
- NOGUCHI N ET AL: "Regulation of transcription of the mph(A) gene for macrolide 2'-phosphotransferase I in Escherichia coli: Characterization of the regulatory gene mphR(A)" JOURNAL OF BACTERIOLOGY, WASHINGTON, DC, US, Bd. 182, Nr. 18, September 2000 (2000-09), Seiten 5052-5058, XP002227127 ISSN: 0021-9193 in der Anmeldung erwähnt
- KURITTU JUSSI ET AL: "Detection of tetracyclines with luminescent bacterial strains." LUMINESCENCE (CHICHESTER), Bd. 15, Nr. 5, September 2000 (2000-09), Seiten 291-297, XP009013811 ISSN: 1522-7235 in der Anmeldung erwähnt

### SEQUENZPROTOKOLL

<110> Bayer Aktiengesellschaft
<120> Verfahren zum Identifizieren von makrocyclischen Polyketiden
<130> Le A 35 526-WO
<140>
   <141>
<150> DE 10138766.0
   <151> 2001-08-07
<160> 4
<170> PatentIn Ver. 2.1
<210> 1
   <211> 36
   <212> DNA
   <213> Künstliche Sequenz
<220>
   <223> Beschreibung der künstlichen Sequenz:Primer
<400> 1
   cgcggatcct gatgcgtgca ctacgcaaag gccagg 36
<210> 2
   <211> 36
   <212> DNA
   <213> Künstliche Sequenz
<220>
   <223> Beschreibung der künstlichen Sequenz:Primer
<400> 2
   ccggaattca gtcagcgggc catggagctt gagccc 36
<210> 3
   <211> 68
   <212> DNA
   <213> Künstliche Sequenz
<220>
   <223> Beschreibung der künstlichen Sequenz:Primer
<400> 3
   tatatagcat gcaagaagga gatatacata tgccccgccc caagctcaag tccgatgacg 60
   aggtactc 68
<210> 4
   <211> 44
   <212> DNA
   <213> Künstliche Sequenz
<220>
   <223> Beschreibung der künstlichen Sequenz:Primer
<400> 4
   tttatagtcg accagggact ctgcacacct ccgtttacgc atgt 44

## Beschreibung

Die vorliegende Erfindung betrifft ein Verfahren zum Identifizieren von makrocyclischen Polyketiden durch ein Biosensor-System, sowie Komponenten dieses Biosensor-Systems.

Makrocyclische Polyketide stellen eine der pharmakologisch wichtigsten Naturstoffklassen dar, da sie sich durch ein breites Wirkspektrum auszeichnen. Sie haben ein polyketidisches Grundgerüst mit mehr als 10 Atomen in einem Ring, der als charakteristisches Merkmal mindestens eine Lacton- oder/und Laktam-Bindung aufweist. Zu den Makroliden, d.h. makrocyclischen Polyketiden mit einem Lactonring, zählen z.B. die antimikrobiell wirksamen und klinisch eingesetzten 14- und 16-gliedrigen Lactone Carbomycin, Erythromycin, Leucomycin oder Oleandomycin. Sie hemmen die Proteinbiosynthese durch Bindung an die 50S-Untereinheit der prokaryotischen 70S-Ribosomen. Daneben gibt es die sog. nicht-klassischen Makrolide, die in der Ringgröße vergleichbar sind, keine Zuckerbausteine aufweisen, aber dennoch biologisch aktiv sind, wie z.B. Soraphen, Albomycin oder das antifungal und cytostatisch aktive Epothilon. Epothilone hemmen ein breites Spektrum von Tumorzell-Linien. Polyen-Makrolide enthalten 20 bis 38-gliedrige Lacton-Ringe und sind häufig mit einem Aminozucker glycosyliert. Sie sind antifungal wirksam und werden z.T. klinisch gegen Pilz- und Hefeinfektionen eingesetzt (z.B. Nystatin). Zu den Spiro-Makroliden zählen die Avermectine und Milbemycine, die gegen Endo- und Ektoparasiten bei Tieren und als Insektizide im Pflanzenschutz einen großen Markt besitzen. Zusätzlich zu dem Lactonring als charakteristischem Merkmal weisen die Pleco-Makrolide eine gefaltete Seitenkette auf. Es handelt sich bei diesen Makroliden um hochaktive ATPase-Inhibitoren, wie z.B. Concanamycin A. Makrodiolide (z.B. Elaiophyllin) und Makrotetrolide (z.B. Nonactin) sind charakteristischer Weise aus repetitiven Untereinheiten aufgebaut. Beispiele für makrocyclische Polyketide, die sowohl eine Lacton- als auch eine Lactam-Bindung aufweisen, sind die strukturell ähnlichen Immunmodulatoren Rapamycin und FK-506. Rifamycin ist ebenfalls ein makrocyclisches Polyketid, wobei keine Lacton-, sondern eine Lactam-Bindung vorliegt. Rifampicin ist ein halbsynthetisches Derivat des Rifamycins mit besseren pharmakologischen Eigenschaften und einem breiteren Wirkungsspektrum.

Nach dem derzeitigen Stand der Technik ist es nicht möglich, makrocyclische Polyketide anhand ihrer charakteristischen strukturellen Eigenschaft in biologischen Screening-Verfahren, z.B. von Naturstoffextrakten, zu erkennen. Man ist vielmehr darauf angewiesen, dass diese Substanzen in einem jeweils eingesetzten Screening-Test, durch die jeweils gesuchte biologische Aktivität auffallen. Makrocyclische Polyketide mit anderer oder anderen, als den im Test gesuchten Wirkungen werden hierdurch nicht erfasst. Aufgrund des außerordentlichen Potentials biologischer Wirksamkeiten makrocyclischer Polyketide ist es jedoch wünschenswert, diese Substanzklasse unabhängig von ihrer Wirkung selektiv zu erkennen.

Eine Möglichkeit zur Detektion von Analyten stellt die Verwendung von Biosensoren dar. Es gibt verschiedene gentechnisch konstruierte, biologische Komponenten eines Biosensors, die ungeachtet der Struktur einer chemischen Substanz selektiv zelluläre Effekte wie Heat-Shock-Aktivierung oder Carcinogene anzeigen. Eine Reihe weiterer biologischer Biosensor-Komponenten kann vornehmlich in Anwesenheit bestimmter Metallsalze oder bestimmter zellulärer Metabolite messbare Signale ausprägen (Daunert et al., 2000). Solche Testsysteme können eine hohe Nachweisempfindlichkeit aufweisen, wie z.B. für Biosensoren zum Nachweis von nitrifikationshemmenden Substanzen (Ludwig et al., 1999) oder Chromatsalzen gezeigt werden konnte (Peitzsch et al., 1998). Ein Biosensor-System für das aromatische Polyketid Tetracyclin ist ebenfalls beschrieben worden (Kurittu et al., 2000). Ein Sensor zur Detektion der pharmakologisch attraktiven Naturstoffklasse der makrocyclischen Polyketide, ist bisher noch nicht beschrieben worden.

Gegenstand der vorliegenden Erfindung ist ein Verfahren zum Identifizieren von makrocyclischen Polyketiden, wobei man makrocyclische Polyketide oder eine Mischung, die makrocyclische Polyketide enthält, einem Biosensor-System aussetzt.

Mit dem erfindungsgemäßen Verfahren unter Anwendung eines Biosensor-Systems wird die Möglichkeit bereitgestellt, ein Screening auf die attraktive Naturstoffklasse der makrocyclische Polyketide zu fokussieren. Da die biologische Aktivität eines makrocyclischen Polyketids für ganz unterschiedliche Bereiche relevant sein kann, wie z.B. für das Gebiet der Gesundheit, der Landwirtschaft oder des Umweltschutzes, kann durch diese fokussierte Vorgehensweise der Aufwand für die Entdeckung neuer biologisch interessanter makrocyclischer Polyketide erheblich vermindert werden.

Unter dem Begriff "Biosensor-System", wie er hierin verwendet wird, wird insbesondere ein System auf der Basis eines gentechnisch hergestellten, zellulären Biosensors verstanden. Der Biosensor besteht aus einer biologischen Komponente, einem mikrobiellen Erkennungssystem, das zur spezifischen Erkennung eines makrocyclischen Polyketids geeignet ist. Die Erkennung des makrocyclischen Polyketids wird durch Stimulierung einer biochemischen Information angezeigt. Diese biochemische Information kann durch eine geeignete technische Komponente gemessen und quantifiziert werden.

Die biologische Komponente des Biosensor-Systems für makrocyclische Polyketide wird durch ein zelluläres Reportergen-Testsystem dargestellt. Ein solches zelluläres Reportergen-Testsystem umfasst besonders bevorzugt ein Reportergen, dessen Transkription unter der Kontrolle einer Promotor-Region steht, welche in Abhängigkeit eines makrocyclischen Polyketids reguliert wird.

Als Reportergene können verschiedene Gene wie z.B. Gene, die für die Chloramphenicol-Acetyltransferase, die Beta-Galactosidase, eine bakterielle oder die Glühwürmchen-Luciferase oder das grün fluoreszierende Protein (GFP) codieren.

Solche Reportergene haben sich bei der Entwicklung von Biosensoren bewährt (Daunert et al., 2000). Bevorzugt wird als Reportergen ein Gen verwendet, welches für Luciferase codiert.

Die von der biologischen Komponente erzeugte biochemische Information kann mit Hilfe von bildgebenden Verfahren, wie photographischen Verfahren, Video Imaging oder auch Handzeichnung festgehalten werden. Die von der biologischen Komponente erzeugte biochemische Information kann auch in ein physikalisch quantifizierbares Signal, unter Verwendung eines Signalwandlers (Transduktors) als technischer Komponente, umgewandelt werden. Dies kann z.B. ein elektrisches Signal sein, das einer elektronischen Verstärkung zugänglich ist. Als Transduktor können z.B. eine amperometrische oder potentiometrische Elektrode, eine Optrode, ein Piezokristall oder ein Thermistor Anwendung finden (Pühler et al., 2000).

Als Promotor, der die Transkription eines Reportergens in Abhängigkeit eines makrocyclischen Polyketids kontrolliert, wird die Promotor-Region des mph(A)-Gens aus E. coli verwendet. Zur Regulation der Promotor-Region wird das MphR(A)-Protein benötigt, welches vorzugsweise durch Überexpression des entsprechenden Gens aus E. coli bereitgestellt wird.

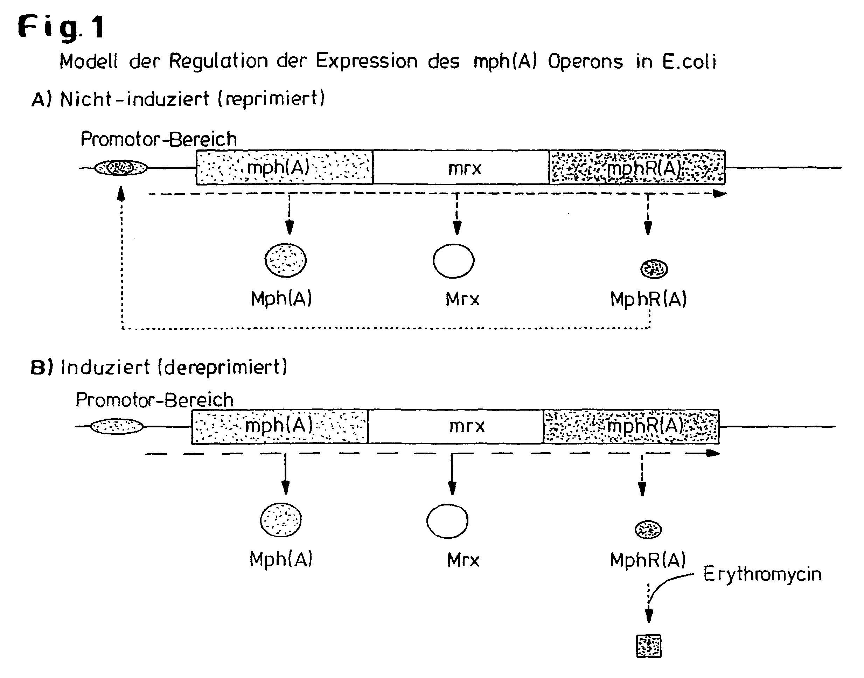

Der biologische Teil des Biosensor-Systems basiert demnach auf Komponenten eines Erythromycin-Resistenzgen-Operons aus E. coli (Noguchi et al., 2000). Entsprechend dem publizierten Modell über dieses mph(A)-Operon werden drei Genprodukte kodiert: Mph(A), Mrx und MphR(A). Hierbei wird die Expression dieses Operons durch Bindung von MphR(A) an die Promotor-Region des mph(A)-Gens, dem ersten Gen dieses Operons, negativ auf Transkriptionsebene reguliert. In Abwesenheit von Erythromycin bindet MphR(A) an die Promotor-Region des mph(A)-Operons und reprimiert dessen Expression. In Anwesenheit von Erythromycin wird die Bindung von MphR(A) an diesen Promotor-Bereich aufgehoben und die Expression des Operons freigegeben (Abb. 1).

Als Wirtszellen, die eine weitere biologische Komponente des Biosensor-Systems für das erfindungsgemäße Verfahren darstellen, können sowohl prokaryotische als auch eukaryotische Zellen verwendet werden. Vorzugsweise werden Bakterien und besonders bevorzugt E. coli, ein Gram-negativer Mikroorganismus verwendet.

Zur Herstellung einer ganz besonders bevorzugten Ausführungsform eines Biosensor-Systems für das erfindungsgemäße Verfahren werden die folgende Komponenten hergestellt und kombiniert: (i) ein Luciferase-Reportergen-Plasmid, dessen Expression unter Kontrolle der mph(A)-Promotor-Region steht, (ii) eine E. coli-Zelle, die das mphR(A)-Gen überexprimiert und gleichzeitig das mph(A)-Operon oder Teile davon trägt.

Die Vorgehensweise bei der Herstellung dieser ganz bevorzugten Ausführungsform wird im Folgenden genauer beschrieben.

Zur Herstellung des oben genannten Luciferase-Reportergen-Plasmids wird beispielsweise die Promotor-Region-codierende und MphR(A)-bindende DNA-Sequenz des mph(A)-Operons des Plasmids pTZ3509 (Nouguchi et al., 2000) durch PCR amplifiziert (vgl. Beispiel 1) und in den Promotor-Suchvektor pUCD615 (Rogowsky et al., 1987) kloniert. Dieser Transkriptionsfusions- (Operonfusions-) Vektor codiert die promotorlosen Luciferase-Strukturgene (lux-Gene) aus Vibrio fischeri. Das Promotor-tragende DNA-Stück wird so in pUCD615 kloniert, dass die Transkription der lux-Gene durch die mph(A)-Promotor-Region kontrolliert wird. Zur Übertragung des mph(A)-Operons, das Resistenz gegenüber Erythromycin verleiht, wird ein mph(A)-Operon codierendes DNA-Fragment aus dem Plasmid pTZ3509 in den Transposon-Suizidvektor pBSL180 (Alexeyev and Shokolenko, 1995) umkloniert (vgl. Beispiel 2). Zur Überexpression des MphR(A)-Proteins wird das mphR(A)-Gen aus dem Plasmid pTZ3509 durch PCR-Amplifikation gewonnen und in den Vektor pACYC184 (Chang and Cohen, 1978) kloniert (vgl. Beispiel 3).

Als E. coli-Zelle wird beispielsweise E. coli SM101 eingesetzt (Vuorio and Vaara, 1992). Es handelt sich bei diesem Stamm um eine lpxA2-Mutante, die einen Defekt in der UDP-N-Acetylglucosamin-Acyltransferase-Aktivität aufweist. Dieses Enzym ist zum ersten Schritt der LipidA-Biosynthese der äußeren Zellmembran nötig. Die lpxA2-Mutation bedingt eine drastische Steigerung der Sensitivität gegenüber hydrophoben Antibiotica wie Erythromycin oder Rifampin.

Das Luciferase-Reportergen-Plasmid, dessen Expression unter Kontrolle der mph(A)-Promotor-Region steht, das mphR(A)-tragende Plasmid sowie das mph(A)-Operon übertragende Suizidplasmid werden durch Standardverfahren der Molekulargenetik in den Bakterienstamm E. coli SM101 übertragen.

Als Wirtszelle kann auch ein E. coli-Stamm verwendet werden, der eine tolC-Mutation trägt. Ein funktionelles tolC-Genprodukt ist für ein wirksames AcrAB-Effluxsystem und zur Expression des Mar-Phänotyps erforderlich (Fralick, 1996). Es spielt möglicherweise eine Rolle beim Ausschleusen von noxischen hydrophoben Substanzen. Selbstverständlich ist es auch ohne weiteres möglich, einen E. coli-Stamm als Wirtsorganismus zur Ausprägung des erfindungsgemäßen Biosensor-Systems einzusetzen, der sowohl eine lpxA2- als auch eine tolC-Mutation trägt.

Durch die beispielhaft vorgestellte Vorgehensweise kann ein Biosensor-System hergestellt werden, welches die Eigenschaft besitzt, dass die Luciferasegen-Expression und damit Lumineszenz durch 12- bis 16- gliedrige makrocyclische Polyketide, stimuliert wird. Beispielsweise zeigt das erfindungsgemäße Biosensor-System die 14-gliedrigen natürlichen makrolidischen Proteinsynthesehemmer Erythromycin, Clarithromycin, Oleandomycin, Pikromycin und Narbomycin an. Das erfindungsgemäße Biosensor-System wird auch durch das 12-gliedrige Makrolid Methymycin, das antibakterielle Wirksamkeit gegenüber Gram-positiven Bakterien hat, zur Lumineszenz angeregt. Darüber hinaus wird das erfindungsgemäße Biosensor-System ebenso durch ein 15-gliedriges, halbsynthetisches Makrolid, das Azithromycin, zur Lumineszenz angeregt. Das makrocyclische Laktam-Polyketid Rifampicin, ein halbsynthetisches Rifamycin-Derivat und Transkriptionshemmer, wird ebenfalls durch das Biosensor-System angezeigt.

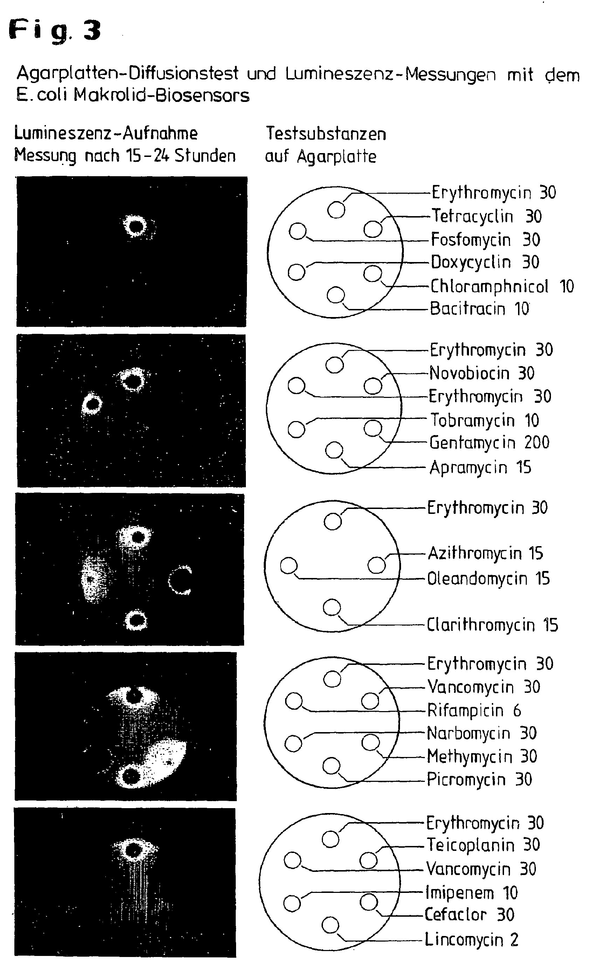

Andere Substanzen, die nicht zur Klasse der makrocyclischen Polyketide gehören, wie beispielsweise die Proteinsynthesehemmer Tetracyclin oder Chloramphenicol, die Zellwandsynthesehemmer Fosfomycin und Vancomycin oder der Gyrasehemmer Novobiocin, führen nicht zu einer solchen Lumineszenz-Anregung (Abb. 3).

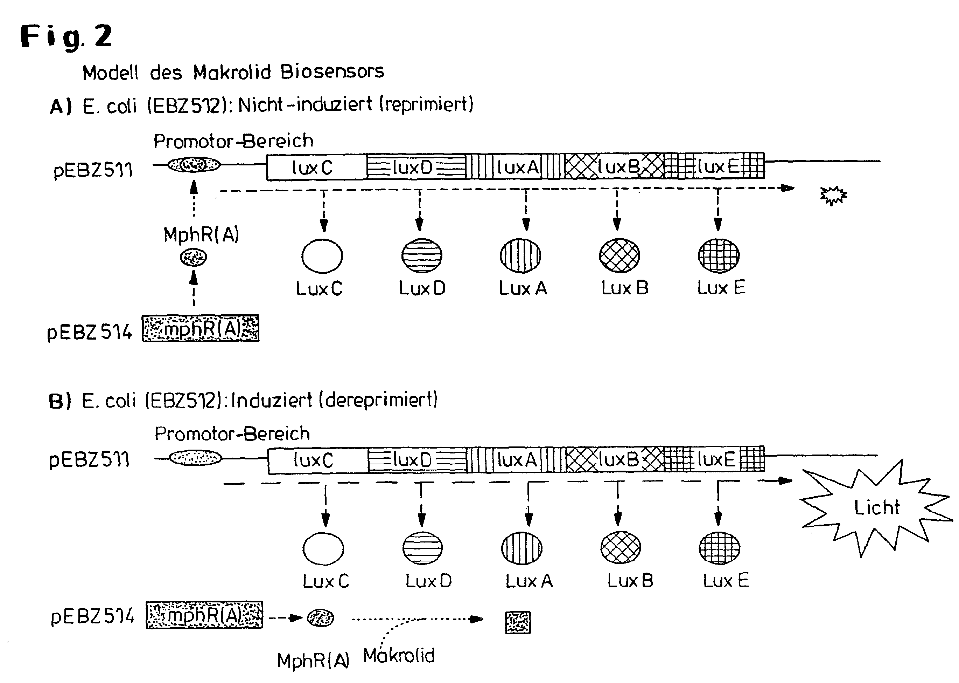

Die Spezifität des erfindungsgemäßen Biosensor-Systems in der ganz bevorzugten Ausführungsform wird entsprechend den vorgestellten Modellen (Abb. 1 und 2) durch den MphR(A)-Repressor und durch dessen Bindung an die mph(A)-Promotor-Region bestimmt. Durch Verfahren wie DNA-Shuffling (Stemmer, 1994), einen Staggered-Extension-Process (Zhao et al., 1998) oder ein neues DNA-Shuffling-Verfahren (Coco et al., 2001) kann die Bindungseigenschaft von MphR(A) moduliert werden. Beispielsweise kann die Bindungsspezifiät oder die Affinität von MphR(A) für bestimmte makrocyclische Polyketide verändert werden. Weiterhin können die DNA-bindenden und Transkriptions-regulierenden Eigenschaften von MphR(A) verändert werden. Ferner kann durch Veränderung der Nukleotidsequenz des DNA-Bereiches, an welche MphR(A) bindet, die Selektivität und Sensitivität des Biosensor-Systems verändert werden. Darüber hinaus ist es möglich, durch Regulierung der zellulären Verfügbarkeit des MphR(A)-Proteins oder anderer Komponenten des Biosensor-Systems, die Sensoreigenschaften zu beeinflussen.

Hieraus ergibt sich ohne Weiteres die Möglichkeit, die biologische Komponente des Biosensor-Systems so zu verändern, dass spezifisch Substanzen erkannt werden können, die von der ganz bevorzugten Ausführungsform nicht spezifisch angezeigt werden. Von besonderem Interesse sind hierbei biologische Biosensor-Komponenten, die z.B. nicht-ribosomal synthetisierte, cyclische Peptide, wie Vankomycin oder Analoga, anzeigen können. Prinzipiell können durch Verfahren wie DNA-Shuffling (s.o.) oder andere mutative Verfahren die Selektivität und Sensitivität des Biosensor-Systems auf eine bestimmte Struktur hin optimiert werden. Bei solchen Veränderung der Biosensor-Eigenschaften kann es z.B. von Vorteil sein, den Promotor des mph(A)-Operons vor promotorlose Gene eines selektierbaren Gens, wie eines Resistenzgens, z.B. eines Tetracyclin-Resistenzgens, zu setzen. Hierdurch können im Zuge von Arbeiten zur Veränderung von Biosensor-Eigenschaften, Varianten des Biosensor-Systems positiv anhand der stimulierbaren Tetracyclin-Resistenz herausgefiltert werden.

Das erfindungsgemäße Biosensor-System kann auch im Rahmen der kombinatorischen Biosynthese eingesetzt werden. So ist es z.B. möglich, das Biosensor-System zu verwenden, um Substanzen einer Polyketid-Bibliothek, wie sie kürzlich beschrieben worden ist (Tang et al., 2001), anzuzeigen. Die Nachweismethode ist unabhängig von der antibakteriellen Aktivität der untersuchten Substanzen. Es ist darüber hinaus auch ohne Weiteres möglich, in einem Biosensor-System der vorliegenden Erfindung ein weiteres System auszuprägen, das eine solche oder ähnliche Polyketid-Bibliothek ausprägt. Hierdurch können Mikroorganismenzellen einfach erkannt werden, die intrazellulär biokombinatorisch erzeugte makrocyclische Polyketide bereitstellen. Wird als Reportergen-Produkt das grün fluoreszierende Protein eingesetzt, können solche Zellen durch ein FACS (Fluoreszenz-aktiviertes Zell-Sortierungs)-Gerät sortiert werden.

Das erfindungsgemäße Biosensor-System hat ein breites Anwendungsspektrum. Es kann z.B. in situ zur Detektion von makrocyclischen Polyketiden in der natürlichen oder antropogenen, belebten oder unbelebten Umwelt eingesetzt werden. Es kann so z.B. zum Nachweis von makrocylischen Polyketide, die von angereicherten oder isolierten Mikroorganismen oder anderen Organismen hergestellt werden, eingesetzt werden (vgl. Beispiel 5). Selbstverständlich kann es auch zum Nachweis nicht nur von natürlichen oder gentechnisch hergestellten, sondern auch von synthetischen makrocyclischen Polyketiden verwendet werden. In besonderer Weise kann es im Labor- oder Produktionsumfeld, in einem homogenen Test im Rahmen von Screening-Verfahren in Reagenzgefäßen oder im Mikrotiterplatten-Format eingesetzt werden. Zudem ist eine Anwendung in einem Agarplatten-Diffusionstest, im Rahmen einer Dünnschichtchromatographie (Shaw et al., 1997; Eberz et al., 1996), im Continous Format High Througput Screening (WO 99/30154) oder im Sensorschicht-Format (DE-A 199 15 310) möglich.

Das erfindungsgemäße Verfahren kann in besonderer Weise unter Anwendung eines Agarplatten-Diffusionstests durchgeführt. Im Fall der Lumineszenz-Anregung ist bereits nach ca. 2 Stunden Inkubationszeit eine Detektion möglich. Im Allgemeinen wird eine über-Nacht-Inkubation (15 bis 24 Stunden) vorteilhaft sein.

Es ist auch ohne Weiteres möglich, die in Weichagar suspendierten Biosensor-Zellen auf einen Bewuchs von Testorganismen, wie z.B. Kolonien zu spreiten, um anhand einer Messung, wie z.B. der Messung der Lumineszenz-Anregung, einen Organismus zu erkennen, der ein makrocyclisches Polyketid produziert.

Anhand der vorliegenden Erfindung ist es auch möglich, mit subzellulären Komponenten des erfindungsgemäßen Biosensor-Systems, z.B. bestimmten Proteinen oder Cofaktoren, einen nicht-zellulären Screening-Test zum Nachweis von makrocyclischen Polyketiden, zu entwickeln.

Gegenstand der vorliegenden Erfindung sind auch die Wirtszellen, die das erfindungsgemäße Biosensor-System ausprägen, sowie entsprechende DNA-Konstrukte.

Weiterhin ist ein Kit, welcher das erfindungsgemäße Biosensor-System enthält, Gegenstand der vorliegenden Erfindung.

### Beispiele

### Beispiel 1

### Protokoll zur Herstellung eines Luciferase-Reporterplasmids

### Herstellung des Plasmids pEBZ511

Zur Herstellung des Plasmids pEBZ511 wurden zunächst der Promotor des *mph(A)-*Operons und die DNA-Sequenz, an die das MphR-Protein bindet durch PCR-Amplifikation gewonnen. Als Templat wurde DNA des Plasmids pTZ3509 (Noguchi et al., 2000) herangezogen. Als Primer wurden die Oligonukleotide mphf216 und mphr217 eingesetzt, die zur Amplifikation des gewünschten DNA-Fragmentes mit einer endständigen BamHI und EcoRI-Erkennungssequenz dienten. Der PCR-Ansatz wurde zunächst 3 Minuten bei 95°C denaturiert und dann 30 Cyclen unterworfen, mit Denaturierung bei 95°C für 60 Sekunden, 58°C für 90 Sekunden zum Annealen, 68°C für 2 Minuten zur Extension. Der PCR-Ansatz enthielt 100 pmol der beiden Primer, 10 ng pTZ3509, 0,5 mM dNTPs, 1 x Pfx Puffer (Life Technologies), 1 x PCR-Enhancer Solution (Life Technologies) und 5 U Pfx-Polymerase (Life Technologies). Das erzielte DNA-Amplifikat wurde isoliert, mit *Bam*HI und *Ec*oRI geschnitten und in den mit BamHI und EcoRI geschittenen Vektor pUCD615 (Rogowsky et al., 1987) mit Standardverfahren der Molekularbiologie (Sambrook et. al., 1989) kloniert.
mphf216: CGCGGATCCTGATGCGTGCACTACGCAAAGGCCAGG
mphr217: CCGGAATTCAGTCAGCGGGCCATGGAGCTTGAGCCC

### Beispiel 2

### Protokoll zur Herstellung eines mph(A)-Operon tragenden Suizid-Vektors

### Herstellung des mph(A)-Operon-tragenden Suizid-Transposonplasmids pEBZ512 und Transposition des mph(A)-Operons in E. coli SM101

Das mph(A)-Operon wurde mit PstI auf einem 4,1 kb großen DNA-Fragment aus dem Plasmid pTZ3509 (Noguchi et al., 2000) herausgetrennt und in die PstI-Schnittstelle des Suizid-Transposon-Vektors pBSL180 (Alexeyev and Shokolenko, 1995), in einen transponierbaren Anteil des Plasmids, mit Standardverfahren der Molekularbiologie kloniert. Als Transformationswirt diente E. coli CC118 Lambdapir (Herrero et al., 1990). Dieser Stamm verfügt über das π-Protein, das zur Replikation des Plasmids nötig ist. Das hergestellte Plasmid erhielt die Bezeichnung pEBZ512. Dieses Plasmid wurde durch Standardverfahren der Transformation nach E. coli SM101 übertragen und die Transformanten nach Transposition des mph(A)-Operon tragenden Transposons selektiert. Das durch diese Vorgehensweise ins Genom integrierte mph(A)-Operon-tragende transponierte DNA-Stück erhielt die Bezeichnung EBZ512. Für die molekularbiologischen Arbeiten (DNA-Isolierungen, Restriktionen, Ligationen und Transformationen wurden Standardverfahren (Sambrook et al., 1989) verwendet.

### Beispiel 3

### Protokoll zur Herstellung eines mphR(A)-codierenden Plasmids

### Herstellung des mphR(A)-codierenden Plasmids pEBZ514

Der Genbereich von mphR(A) wurde mit Hilfe der Primer MLV637 und MLV638 und pEBZ512-DNA mittels PCR amplifiziert. In Primer MLV637 wurde eine Ribosomenbindestelle und eine SphI-Schnittstelle, in Primer MLV638 eine SalI-Schnittstelle integriert. Die PCR-Reaktion wurde auf einem Eppendorf Thermocycler Modell Mastercycler Gradient durchgeführt. Der PCR-Ansatz wurde zuerst 10 Minuten bei 96°C denaturiert und anschließend 30 Elongationscyclen unterworfen, mit Denaturierung bei 96°C, 60 Sekunden, Annealing bei 56°C, 90 Sekunden und Elongation bei 72°C, 40 Sekunden. Der PCR-Ansatz enthielt 100 pmol der beiden Primer, 10 ng pEBZ512-DNA, 0,5 mM dNTPs, 1 x Pfx Puffer (Life Technologies), 1 x PCR-Enhancer Solution (Life Technologies) und 5 U Pfx-Polymerase (Life Technologies). Das entstandene DNA-Fragment wurde isoliert, mit den Restriktionsenzymen SphI und SalI geschnitten und in den ebenfalls mit SphI und SalI geschnittenen Vektor pACYC184 (Chang und Cohen, 1978) ligiert. Die mphR(A) codierende Region lag somit innerhalb des Tetracyclinresistenzgens, hinter dem zugehörigen Resistenzgen-Promotor. Für die molekularbiologischen Arbeiten (DNA-Isolierungen, Restriktionen, Ligationen und Transformationen wurden Standardverfahren (Sambrook et al., 1989) verwendet.
MLV637:
MLV638:
   TTTATAGTCGACCAGGGACTCTGCACACCTCCGTTTACGCATGT

### Beispiel 4

### Lumineszenz-Messungen im Agarplatten-Diffusionstest

Der Biosensor E. coli SM101 (EBZ512, pEBZ511, pEBZ514) wurde in LB-Medium aerob bis zur spät-logarithmischen Wachstumgsphase angezogen, in Weichagar unter Standardverfahren der Mikrobiologie eingemischt und auf LB-Agarplatten, die Chloramphenicol (25µg/ml) und Kanamycin (30µg/ml) enthielten, ausgespreitet. Nach Erstarren des Weichagars wurden Testplättchen zur Empfindlichkeitsprüfung, die die zu testende Substanz bereits enthielten oder unbeschickte Testblättchen (Oxoid GmbH, Am Lippeglacis 4-8, 46483 Wesel, Deutschland), auf die die zu testende Substanz appliziert wurde, aufgelegt. Der Agarplatten-Diffusionstest wurde bei 28°C für 2 bis 4 Stunden oder 15 bis 24 Stunden bebrütet und die Lumineszenz mit einem Berthold Luminograph LB980 aufgezeichnet (Abb.3, 4 und 5). Bei Auftreten einer Lumineszenz-Anregung ist diese im Bereich einer Diffusionszone um die aufgelegte Probe zu erkennen. Hat die getestete Probe gleichzeitig eine auf den Sensor-Organismus antibakteriell wirksame Aktivität, die sich bei der ganz bevorzugten Ausführungsform der längeren Inkubation als Hemmhof bemerkbar macht, so ist die Lumineszenz-Anregung außerhalb dieses Hemmhofrings im Bereich subletaler Konzentrationen zu erkennen.

### Beispiel 5

### Detektion eines Makrolid- (Erythromycin-) bildenden Organismus

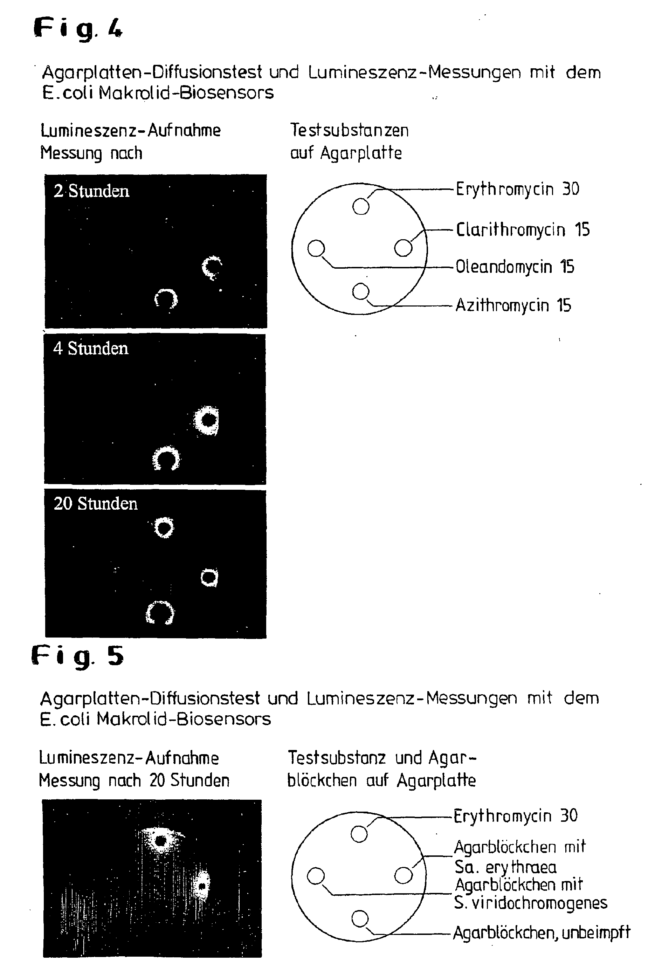

Mit dem Biosensor war es auch möglich, einen Mikroorganismus, der als Produzent eines makrocyclischen Polyketids bekannt ist, in situ von einem anderen Mikroorganismus zu unterscheiden. Hierzu wurde die mikrobielle Biosensorzelle im Agarplatten-Diffusionstest eingesetzt. Auf der in Weichagar eingebetteten Biosensorsuspension wurden Agarblöckchen gesetzt. Diese Agarblöckchen waren entweder mit Saccharopolyspora erythraea DSM 40517, der als Erythromycin-Produzent beschrieben ist, oder mit Streptomyces viridochromogenes DSM 40721, der als Avilamycin-Produzent beschrieben ist, bewachsen. Ein weiteres Agarblöckchen war unbewachsen. Eine Lumineszenz-Anregung war nur bei dem zusätzlich aufgelegten Erythromycin-Testblättchen und dem mit Sa. erythraea bewachsenen Agarblöckchen festzustellen (Abb. 5).

### Beschreibung der Abbildungen:

### Abbildung 1

Modell der Regulation des mph(A)-Operons in E. coli (nach Noguchi et al., 2000, modifiziert)

### Abbildung 2

Modell der ganz bevorzugten Ausführungsform der biologischen Komponente des Biosensor-Systems. luxC, D, A, B, E und LuxC, D, A, B und E, Luciferase-Strukturgene bzw. Luciferase-Strukturgenprodukte aus Vibrio fischeri. mphR(A), MphR(A), siehe Noguchi et al., 2000.

### Abbildung 3

Agarplatten-Diffusionstest und Luminezenz-Messungen mit dem E. coli Biosensor-System. Darstellung der Selektivität der biologischen Sensor-Komponente. Die Zahlenwerte bei den Angaben der Testsubstanzen geben die applizierten Substanzmengen in µg an.

### Abbildung 4

Agarplatten-Diffusionstest und Luminezenz-Messungen mit dem E. coli Biosensor-System. Beispielhafte Darstellung einer zeitlichen Abhängigkeit der Lumineszenz-Anregung der biologischen Sensor-Komponente. Die Zahlenwerte bei den Angaben der Testsubstanzen geben die applizierten Substanzmengen in µg an.

### Abbildung 5

Agarplatten-Diffusionstest und Luminezenz-Messungen mit dem E. coli Biosensor-System. In situ Lumineszenz-Anregung durch eine Erythromycin-bildende Saccharopolyspora erythraea-Kultur. Der Zahlenwert bei der Angabe der Referenzsubstanz Erythromycin gibt die applizierte Substanzmenge in µg an.

### Hinterlegungen

Folgende Stämme sind bei der Deutschen Sammlung von Mikroorganismen und Zellkulturen GmbH (DSMZ), Mascheroder Weg 1b, D-38124 Braunschweig, Deutschland, am 6. Juni 2001 in Übereinstimmung mit den Bestimmungen des Budapester Vertrages hinterlegt worden:

| Stamm-Bezeichnung | Hinterlegungs-Nr. |
|---|---|
| E. coli SM101 (EBZ512, pEBZ511, pEBZ514) | DSM 14334 |
| E. coli SM101 (EBZ512) | DSM 14333 |

### Literatur

Alexeyev M., and Shokolenko I.N. (1995) Mini-Tn10 Transposon Derivatives for Insertion Mutagenesis and Gene Delivery into the Chromosome of Gram-negative Bacteria. Gene 160: 59-62.
Chang A.C.Y., and Cohen S.N. (1978) Construction and Characterization of Amplifiable Multicopy DNA Cloning Vehicles Derived from the P15A Cryptic Miniplasmid. J. Bacteriol. 134: 1141-1156.
Coco W.M., Levinson W.E., Crist M.J., Hektor H.J., Darzins A., Pienkos P.T., Squires C.H., and Monticello D.J. (2001) DNA shuffling method for generating highly recombined genes and evolved enzymes. Nature Biotechnology 19: 354-359.
Daunert S., Barrett G., Feliciano J.S., Shetty R.S., Shrestha S., and Smith-Spencer W. (2000) Genetically Engineered Whole-Cell Sensing Sytems: Coupling Biological Recognition with Reporter Genes. Chem. Rev. 100: 2705-2738.
Eberz G., Rast H.G., Burger K., Kreiss W., Weisemann C. (1996) Bioactivity screening by chromatography-bioluminescence coupling. Chromatographia 43: 5-9.
Fralick J.A. (1996) Evidence that TolC is Required for Functioning of the Mar/AcrAB Efflux Pump in Escherichia coli. J. Bacteriol. 178: 5803-5805.
Herrero M., de Lorenzo V., and Timmis K.N. (1990) Transposon Vectors Containing Non-Antibiotic Resistance Selection Markers for Cloning and Stable Chromosomal Insertion of Foreign Genes in Gram-negative Bacteria. J. Bacteriol. 172: 6557-6567.
Kurittu J., Karp M., and Korpela M. (2000) Detection of Tetracyclines with Luminescent Bacterial Strains. Luminescence 15: 291-297.
Ludwig C., Ecker S., Schwindel K., Rast H.G., Stetter K.O., and Eberz G. (1999) Construction of Highly Bioluminescent Nitrosomonas as a Probe for Nitrification Conditions. Arch. Microbiol. 172: 45-50.
Noguchi N., Takada K., Katayama J., Emura A., and Sasatsu M. (2000) Regulation of Transcription of mph(A) Gene for Macrolide 2'-Phosphotransferase I in Escherichia coli: Characterization of the Regulatory Gene mphR(A). J. Bacteriol. 182: 5052-5058.
Peitzsch N., Eberz G., and Nies D.H. (1998) Alcaligenes eutrophus as a Bacterial Chromate Sensor. Appl. Environ. Microbiol. 64: 453-458.
Pühler A., Regitz M. und Schmid R.D. (2000) Biochemie und Molekularbiologie. Römpp Lexikon, Georg Thieme Verlag Stuttgart - New York.
Rogowsky P.M., Close T.J., Chimera J.A., Shaw J.J., and Kado C.I. (1987) Regulation of the vir Genes of Agrobacterium tumefaciens Plasmid pTiC58. J. Bacteriol. 169: 5101-5112.
Sambrook J., Fritsch E.F., Maniatis T. (1989) Molecular Cloning: A Laboratory Manual. Cold Spring Harbor Laboratory Press.
Shaw P.D., Ping G., Daly S.L., Cha C., Cronan J.E., Rinehart K.L., and Farrand S.K. (1997) Detecting and Characterzing N-acyl-homoserine Lactone Signal Molecules by Thin-layer-chromatography. Proc. Natl. Acad. Sci. USA 94: 6036-6041.
Stemmer W.P.C. (1994) DANN Shuffling by Random Fragmentation and Reassembly - in-vitro Recombination for Molecular Evolution. Proc. Nat. Acad. Sci. 91: 10747-10751.
Tang L., McDaniel R. (2001) Construction of Desosamine Containing Polyketide Libraries using a glycosyltransferase with broad substrate specificity. Chem. Biol. 8: 547-555.
Vuorio R., and Vaara M. (1992) The Lipid A Biosynthesis Mutations lpxA2 of Escherichia coli Results in Drastic Antibiotic Supersusceptibility. Antimicrob. Agents Chemother. 36: 826-829.
Zhao H.M., Giver L., Shao Z.X., Affholter J.A., and Arnold F.H. (1998) Molecular Evolution by Staggered Extension Process (StEP) in vitro Recombination. Nature Biotechnology 16: 258-261.

## Patentansprüche

1. Verfahren zum Identifizieren von makrocyclischen Polyketiden, **dadurch gekennzeichnet, dass** man makrocyclische Polyketide oder eine Mischung, die makrocyclische Polyketide enthält, einem zellulären Reportergen-Testsystem aussetzt, welches ein Reportergen, dessen Transkription unter der Kontrolle der Promotor-Region des mph(A)-Gens aus E. coli steht, und das MphR(A)-Protein von E. coli umfasst.

2. Verfahren gemäß Anspruch 1, **dadurch gekennzeichnet, dass** es sich bei den makrocyclischen Polyketiden um Makrolide handelt.

3. Verfahren gemäß Anspruch 1 oder 2, **dadurch gekennzeichnet, dass** als Reportergen ein Gen eingesetzt wird, welches für Chloramphenicol-Acetyltransferase, Beta-Galactosidase, Luciferase oder das grün fluoreszierende Protein (GFP) codiert.

4. Verfahren gemäß Anspruch 3, **dadurch gekennzeichnet, dass** als Reportergen ein Gen eingesetzt wird, welches für Luciferase codiert.

5. Verfahren gemäß einem der Ansprüche 1 bis 4, **dadurch gekennzeichnet, dass** das MphR(A)-Protein durch Überexpression des mphR(A)-Gens bereitgestellt wird.

6. Verfahren gemäß einem der Ansprüche 1 bis 5, **dadurch gekennzeichnet, dass** das Testsystem das gesamte mph(A)-Operon aus E. coli oder Teile davon umfasst.

7. Verfahren gemäß einem der Ansprüche 1 bis 6, **dadurch gekennzeichnet, dass** es sich bei den Zellen um E. coli handelt.

8. Verfahren gemäß Anspruch 7, **dadurch gekennzeichnet, dass** es sich um E. coli SM 101 (EBZ512, pEBZ511, pEBZ514) hinterlegt unter DSM 14334 handelt.

9. Verfahren gemäß Anspruch 7, **dadurch gekennzeichnet, dass** es sich bei E. coli um einen Stamm handelt, welcher eine Mutation im lpxA2- und/oder tolC-Gen besitzt.

10. Wirtszelle umfassend ein Reportergen, dessen Transkription unter der Kontrolle der Promotor-Region des mph(A)-Gens aus E. coli steht, und das MphR(A)-Protein von E. coli.

11. Wirtszelle gemäß Anspruch 10, **dadurch gekennzeichnet, dass** das Reportergen ein Gen darstellt, welches für Chloramphenicol-Acetyltransferase, Beta-Galactosidase, Luciferase oder das grün fluoreszierende Protein (GFP) codiert.

12. Wirtszelle gemäß Anspruch 11, **dadurch gekennzeichnet, dass** das Reportergen ein Gen darstellt, welches für Luciferase codiert.

13. Wirtszelle gemäß einem der Ansprüche 10 bis 12, **dadurch gekennzeichnet, dass** das MphR(A)-Protein durch Überexpression des mphR(A)-Gens bereitgestellt wird.

14. Wirtszelle gemäß einem der Ansprüche 10 bis 13, **dadurch gekennzeichnet, dass** sie das gesamte mph(A)-Operon aus E. coli umfasst.

15. Wirtszelle gemäß einem der Ansprüche 10 bis 14, **dadurch gekennzeichnet, dass** es sich um E. coli handelt.

16. Wirtszelle gemäß Anspruch 15, **dadurch gekennzeichnet, dass** es sich um E. coli SM 101 (EBZ512, pEBZ511, pEBZ514) hinterlegt unter DSM 14334 handelt.

17. Wirtszelle gemäß Anspruch 15, **dadurch gekennzeichnet, dass** es sich bei E. coli um einen Stamm handelt, welcher eine Mutation im lpxA2- und/oder tolC-Gen besitzt.

18. Verwendung einer Wirtszelle gemäß einem der Ansprüche 10 bis 17 zum Identifizieren von makrocyclischen Polyketiden.

19. DNA-Konstrukt umfassend ein Reportergen mit der Promotor-Region des mph(A)-Gens aus E. coli.

20. DNA-Konstrukt gemäß Anspruch 19, **dadurch gekennzeichnet, dass** das Reportergen ein Gen darstellt, welches für Chloramphenicol-Acetyltransferase, Beta-Galactosidase, Luciferase oder das grün fluoreszierende Protein (GFP) codiert.

21. DNA-Konstrukt gemäß Anspruch 20, **dadurch gekennzeichnet, dass** das Reportergen ein Gen darstellt, welches für Luciferase codiert.

22. Kit enthaltend eine Wirtszelle gemäß einem der Ansprüche 10 bis 17.

## Claims

1. Method for identifying macrocyclic polyketides, **characterized in that** macrocyclic polyketides or a mixture containing macrocyclic polyketides are subjected to a cellular reporter-gene assay system which comprises a reporter gene whose transcription is under the control of the promoter region of the E. coli mph(A) gene, and comprises the E. coli MphR(A) protein.

2. Method according to Claim 1, **characterized in that** the macrocyclic polyketides are macrolides.

3. Method according to Claim I or 2, **characterized in that** the reporter gene used is a gene coding for chloramphenicol acetyltransferase, beta-galactosidase, luciferase or green fluorescent protein (GFP).

4. Method according to Claim 3, **characterized in that** the reporter gene used is a gene coding for luciferase.

5. Method according to any of Claims 1 to 4, **characterized in that** the MphR(A) protein is provided by overexpressing the mphR(A) gene.

6. Method according to any of Claims 1 to 5, **characterized in that** the assay system comprises the complete E. coli mph(A) operon or parts thereof.

7. Method according to any of Claims 1 to 6, **characterized in that** the cells are E. coli.

8. Method according to Claim 7, **characterized in that** they are E. coli SM 101 (EBZ512, pEBZ511, pEBZ514), deposited under DSM 14334.

9. Method according to Claim 7, **characterized in that** E. coli is a strain having a mutation in the lpxA2 gene and/or the tolC gene.

10. Host cell comprising a reporter gene whose transcription is under the control of the promoter region of the E. coli mph(A) gene, and comprising the E. coli MphR(A) protein.

11. Host cell according to Claim 10, **characterized in that** the reporter gene is a gene coding for chloramphenicol acetyltransferase, beta-galactosidase, luciferase or green fluorescent protein (GFP).

12. Host cell according to Claim 11, **characterized in that** the reporter gene is a gene coding for luciferase.

13. Host cell according to any of Claims 10 to 12, **characterized in that** the MphR(A) protein is provided by overexpressing the mphR(A) gene.

14. Host cell according to any of Claims 10 to 13, **characterized in that** it comprises the complete E. coli mph(A) operon.

15. Host cell according to any of Claims 10 to 14, **characterized in that** it is E. coli.

16. Host cell according to Claim 15, **characterized in that** it is E. coli SM 101 (EBZ512, pEBZ511, pEBZ514), deposited under DSM 14334.

17. Host cell according to Claim 15, **characterized in that** E. coli is a strain having a mutation in the lpxA2 gene and/or the tolC gene.

18. Use of a host cell according to any of Claims 10 to 17 for identifying macrocyclic polyketides.

19. DNA construct comprising a reporter gene having the promoter region of the E. coli mph(A) gene.

20. DNA construct according to Claim 19, **characterized in that** the reporter gene is a gene coding for chloramphenicol acetyltransferase, beta-galactosidase, luciferase or green fluorescent protein (GFP).

21. DNA construct according to Claim 20, **characterized in that** the reporter gene is a gene coding for luciferase.

22. Kit comprising a host cell according to any of Claims 10 to 17.

## Revendications

1. Méthode d'identification de polycétides macrocycliques, **caractérisée en ce qu'**on soumet des polycétides macrocycliques ou un mélange qui contient des polycétides macrocycliques à un système d'épreuve cellulaire à gène rapporteur qui comprend un gène rapporteur dont la transcription est sous le contrôle de la région promoteur du gène mph(A) de E. coli, et la protéine MphR(A) de E. coli.

2. Méthode suivant la revendication 1, **caractérisée en ce que** les polycétides macrocycliques sont des macrolides.

3. Méthode suivant la revendication 1 ou 2, **caractérisée en ce qu'**on utilise comme gène rapporteur un gène qui code l'acétyltransférase du chloramphénicol, la bêta-galactosidase, la luciférase ou la protéine à fluorescence verte (GFP).

4. Méthode suivant la revendication 3, **caractérisée en ce qu'**on utilise comme gène rapporteur un gène qui code la luciférase.

5. Méthode suivant l'une des revendications 1 à 4, **caractérisée en ce que** la protéine MphR(A) est mise à disposition par surexpression du gène mphR(A).

6. Méthode suivant l'une des revendications 1 à 5, **caractérisée en ce que** le système d'épreuve comprend l'opéron mph(A) entier de E. coli ou des parties de cet opéron.

7. Méthode suivant l'une des revendications 1 à 6, **caractérisée en ce que** les cellules consistent en E. coli.

8. Méthode suivant la revendication 7, **caractérisée en ce qu'**il s'agit de E. coli SM 101 (EBZ512, pEBZ511, pEBZ514) déposé sous la référence DSM 14334.

9. Méthode suivant la revendication 7, **caractérisée en ce qu'**il s'agit d'une souche de E. coli qui possède une mutation dans le gène lpxA2 et/ou dans le gène tolC.

10. Cellule hôte comprenant un gène rapporteur dont la transcription est sous le contrôle de la région promoteur du gène mph(A) de E. coli et la protéine MphR(A) de E. coli.

11. Cellule hôte suivant la revendication 10, **caractérisée en ce que** le gène rapporteur représente un gène qui code l'acétyltransférase du chloramphénicol, la bêta-galactosidase, la luciférase ou la protéine à fluorescence verte (GFP).

12. Cellule hôte suivant la revendication 11, **caractérisée en ce que** le gène rapporteur représente un gène qui code la luciférase.

13. Cellule hôte suivant l'une des revendications 10 à 12, **caractérisée en ce que** la protéine MphR(A) est mise à disposition par surexpression du gène mphR(A).

14. Cellule hôte suivant l'une des revendications 10 à 13, **caractérisée en ce qu'**elle comprend l'opéron mph(A) entier de E. coli.

15. Cellule hôte suivant l'une des revendications 10 à 14, **caractérisée en ce qu'**il s'agit de E. coli.

16. Cellule hôte suivant la revendication 15, **caractérisée en ce qu'**il s'agit de E. coli SM 101 (EBZ512, pEBZ511, pEBZ514) déposé sous la référence DSM 14334.

17. Cellule hôte suivant la revendication 15, **caractérisée en ce qu'**il s'agit d'une souche de E. coli qui possède une mutation dans le gène lpxA2 et/ou dans le gène tolC.

18. Utilisation d'une cellule hôte suivant l'une des revendications 10 à 17 pour l'identification de polycétides macrocycliques.

19. Produit d'assemblage d'ADN comprenant un gène rapporteur doté de la région promoteur du gène mph(A) de E. coli.

20. Produit d'assemblage d'ADN suivant la revendication 19, **caractérisé en ce que** le gène rapporteur représente un gène qui code l'acétyltransférase du chloramphénicol, la bêta-galactosidase, la luciférase ou la protéine à fluorescence verte (GFP).

21. Produit d'assemblage d'ADN suivant la revendication 20, **caractérisé en ce que** le gène rapporteur représente un gène qui code la luciférase.

22. Kit contenant une cellule hôte suivant l'une des revendications 10 à 17.
